# EUROPEAN PATENT APPLICATION

(11) **EP 4 840 962 A1**
(43) Date of publication of application: **18.11.2026**
(21) Application number: 25176751.3
(22) Date of filing: 15.05.2025
(51) Int. Cl.: C12N 15/70

(54) **BIOCONVERSION OF REDUCED C1 COMPOUNDS**

(71) Applicant: ETH Zurich, 8092 Zurich (CH)
(72) Inventor: KELLER, Philipp, 6340 Baar (CH); JAEGER, Benedikt Georg, 8050 Zurich (CH); VORHOLT, Julia Anne, 8044 Zurich (CH)
(74) Representative: P&TS SA (AG, Ltd.)

(57) **Abstract**

The invention concerns a recombinant non-naturally occurring methanol-dependent or fully methylotrophic microorganism, which heterologously expresses or over-expresses a polypeptide having methanol dehydrogenase (Mdh) activity, wherein the initial maximum velocity of the oxidization of formaldehyde to formate reaction (VₘₐₓCH₂O) catalysed by said polypeptide having Mdh activity measured as [nM_{NADH} second⁻¹ µM_{enzyme}⁻¹], is less than 1, or less than 0.1 or 0. The invention also concerns a recombinant Mdh polypeptides and a method for producing microbial metabolites and products from C1 compounds, such as methanol.

## Description

### Technical domain

The present invention concerns recombinant microorganisms converting methanol into carbon metabolites.

### Related art

The release of greenhouse gases (GHG) from anthropogenic sources over the last two centuries has caused a global climate crisis, driving the need for sustainable approaches to mitigate GHG emissions. Achieving a zero-emission chemical industry requires the replacement of fossil feedstocks with CO₂-based alternatives. Among these, the one-carbon compound methanol is an attractive sustainable feedstock because it can be produced renewably from GHG (e-methanol) or biomass (bio-methanol) at scale and at competitive costs.

Biological cell factories have attracted increasing interest for converting methanol into a wide range of value-added products. Natural methylotrophs, which have been studied intensively over many decades, have been used and engineered for methanol-based production. Substantial progress has been made; however, they have not yet been adopted in large-scale industrial processes.

As an alternative approach, four independently engineered and evolved *Escherichia coli* strains which are capable of growing on methanol as sole carbon source have been reported as of today. These reports are particularly promising, as *E. coli* is a well-established and readily scalable species widely used in biological fermentation and production processes.

Kim S. et al. (Nat. Chem. Biol. (2020), DOl:10.1038/s41589-020-0473-5) for example describes a genetically altered *E. coli* strain, which metabolises methanol through the reductive glycine pathway. Additional heterologous expression of a methanol dehydrogenase rendered this strain capable of growing on methanol.

WO2018148703A1 discloses a method for increasing production of a metabolite by a non-naturally occurring methylotroph when grown on a medium comprising methanol. Methanol is metabolised using a heterologous methanol dehydrogenase and the ribulose monophosphate (RuMP) cycle.

WO2024018304A1 and Keller et al (Nat. Comm. (2022), DOI: 10.1038/s41467-022-32744-9) disclose recombinant fully methylotrophic *E. coli* strains, capable of converting methanol and other C1 compounds into value added microbial products through the RuMP cycle.

Chen F.Y-H. et al (Cell (2020), DOI:10.1016/j.cell.2020.07.010) reported formate formation when methanol is metabolized through the RuMP cycle in a synthetic methanol-dependent strain.

Meng et al. (Nat. Comm. (2025), DOI: 10.1038/s41467-024-55502-5) have recently described another synthetic methanol assimilation pathway for *E.coli.*

All of the genetically engineered methylotrophic *E. coli* strains described to date channel formaldehyde generated from enzymatic methanol oxidation into its central carbon metabolism. To allow for an economic and efficient methanol-based bioproduction, methanol should preferably be channelled exclusively into the desired product with minimal generation of unwanted by-products.

Efficiency however remains a major challenge today, in particular since recombinant methylotrophic *E. coli* strains have been found to accumulate significant amounts of formate, in some cases over 10 mM, as an unwanted side-product during aerobic growth. Formate may for example be formed via *E. coli's* native glutathione-dependent formaldehyde detoxification pathway. However, in synthetic methylotrophy, this detoxification pathway is commonly inactivated to redirect carbon flux towards biomass. Deactivation of this pathway is usually achieved by deleting the *frmA* gene coding for the S-(hydroxymethyl)glutathione dehydrogenase or the entire *frmRAB* operon. Gene deletions have either been introduced by genetic engineering, or have been observed during evolution experiments. Formate production in these strains thus appears to occur independently from this detoxification pathway.

Formate as a side product becomes toxic to bacteria when present above 50 mM and probably also at even lower concentrations. In addition, formic acid formed in the growth supernatant is corrosive and reduces the pH of the growth medium. This unwanted formate side-production thus severely hampers biomass production and reduces product yield.

Moreover, since formate cannot be assimilated by the recombinant methylotrophic *E. coli* strains, its carbon is lost, which in turn further lowers product yield. As a further drawback, the accumulated formate contaminates metabolites which are produced using these strains.

It is therefore highly desirable to reduce the production of formate in the microbial bioconversion of methanol, thereby increasing the efficiency of converting methanol into biomass or into value-added microbial products.

### Short disclosure of the invention

It is an aim of the present invention to provide recombinant methylotrophic microorganisms, which overcome the shortcomings and limitations of the state of the art.

It is another aim of the invention to reduce the amount of formate formed during the bioconversion of methanol into biomass or into value added microbial products.

It is another aim of this invention to render the bioconversion of C1 compounds to value-added chemicals more efficient.

It is yet another aim of this invention to provide an alternative recombinant methylotrophic microorganism suitable for industrial bioconversion of methanol.

According to the invention, one or more of these aims are attained by the object of the attached claims, and especially by the independent claims. Further optional embodiments are provided in the dependent claims.

In particular, one or more of these aims are attained by a recombinant non-naturally occurring methylotrophic microorganism, which heterologously expresses or over-expresses a polypeptide having methanol dehydrogenase (Mdh) activity, wherein the maximum velocity of the oxidization of formaldehyde to formate reaction (VₘₐₓCH₂O) catalysed by said polypeptide measured as [nM_{NADH} second⁻¹ µM_{enzyme}⁻¹], is less than 1, or less than 0.1 or 0.

The Mdh activity is determined *in vitro* using the Mdh enzyme assay utilizing the absorption change of the solution at 340nm to quantify NADH and with this indirectly also formaldehyde for the reaction from methanol to formaldehyde or formate for the reaction from formaldehyde to formate.

The recombinant non-naturally occurring methylotrophic microorganism may express a polypeptide having 3-hexulose-6-phosphate synthase (Hps) activity, and polypeptide having a 6-phospho 3-hexuloisomerase (Phi) activity.

In one embodiment, the microorganism either does not comprise, or comprises deletions or reductions of expression of, or eliminations or reductions of activity of a polypeptide having triose-phosphate-isomerase activity and of a polypeptide having glutathione-dependent formaldehyde dehydrogenase activity.

The microorganism may be a synthetic methylotroph comprising one or more of the deletions or point mutations identified by Keller et al., Nat. Comm. (2020), DOI: 10.1038/s41467-020-19235-5.

The maximum velocity of the oxidization of methanol to formaldehyde (Vₘₐₓ CH₃OH) measured as [nM_{NADH} second⁻¹ µM_{enzyme}⁻¹] catalysed by said polypeptide having Mdh activity may be at least 25 times higher, or at least 50 times higher, or at least 100 times higher than the maximum velocity of the oxidization of formaldehyde to formate reaction (VₘₐₓCH₂O), measured as [nM_{NADH} second⁻¹ µM_{enzyme}⁻¹] catalysed by said polypeptide having Mdh activity.

The first metabolic step shared by all natural methylotrophic bacteria for growth on methanol is its oxidation to formaldehyde.

All synthetically engineered methylotrophic *E. coli* strains described to date employ NAD-dependent Mdh as the methanol-oxidizing enzyme. The choice of an NAD-dependent enzyme is justified by its ease of expression in *E. coli* and by the cell's ability to readily utilize NADH in its electron transport chain.

To channel formaldehyde generated from methanol oxidation into central carbon metabolism, the ribulose monophosphate (RuMP) cycle was introduced into recombinant methylothrophic *E. coli* strains. This highly efficient pathway requires the heterologous expression of only two additional genes, 3-hexulose 6-phosphate synthase *(hps)* and 6-phospho 3-hexulosiomerase (*phi*)*.* In this pathway, formaldehyde condenses with ribulose 5-phosphate to form hexulose 6-phosphate, thereby directly integrating the toxic intermediate into biomass and potential products.

To render bioproduction from methanol as efficient as possible, a maximum amount of the formaldehyde needs to enter the RuMP metabolic cycle and losses, for example in the form of formate, which cannot enter the RuMP cycle, should be avoided.

By providing for an enzyme having Mdh activity which oxidizes methanol to formaldehyde, but which is not able or only has a limited capacity to oxidize formaldehyde to formate, this loss of C1 compound for the central metabolism of the methylotrophic microbe is avoided. Thus, the Mdh polypeptide used in this invention channels the assimilated C1 compound into the central metabolism while avoiding wasteful production of formate.

VₘₐₓCH₂O, the maximum velocity of the reaction catalysed by said polypeptide having Mdh activity, measured as [nM_{NADH} second⁻¹ µM_{enzyme}⁻¹] as determined *in vitro* using the protocols described in the Methods section below, is less than 1, or less than 0.1 or 0.

When VₘₐₓCH₂O is assessed in *in vivo* experiments, these values may be higher, as formate production may also arise from metabolic sources within the recombinant microorganism other than from the heterologously expressed Mdh enzyme.

Synthetic methylotrophic *Escherichia coli* strains utilizing the RuMP cycle described today produce substantial amounts of formate under aerobic growth conditions, which was confirmed by this invention.

Results of this invention demonstrated that the formate build-up is due to the inherent property of NAD-dependent methanol dehydrogenase Mdh2 from *Cupriavidus necator* to overoxidize methanol to formate and showed that this feature is widespread among NAD-dependent Mdhs of the overoxidizing class and derivatives thereof.

Moreover, it was found that *Bacillus methanolicus* Mdhs, previously grouped as Mdh/Mdh1 class enzymes based on sequence analysis, do not overoxidize methanol but catalyze the oxidation of methanol to formaldehyde but not formate. As formaldehyde, but not formate, is assimilated via the ribulose monophosphate (RuMP) pathway, these findings explain the physiological importance of Mdh/Mdh1 paralogs in natural methylotrophs and highlight the need to select appropriate Mdh variants for synthetic methylotrophy.

In one embodiment, the Mdh polypeptide is therefore selected from the phylogenetic Mdh/Mdh1 class of Mdh enzymes. These non-overoxidizing enzymes, which are not oxidizing methanol to formate but merely to formaldehyde, are highly similar to each other and form a distinct phylogenetic class with at least 30% difference in amino acid sequence than any other Mdh.

In one embodiment the polypeptide having a Mdh activity has at least 70%, 75%, 80%, 85%, 90%, 95%, 98% or 99% sequence identity to the Mdh Bm MGA3 polypeptide (SEQ ID NO 1).

The Mdh/Mdh1 class comprises the following members, which have either been described previously or identified in the National Center for Biotechnology Information (NCBI) database:
Mdh Bm MGA 3 (SEQ ID NO 1), Mdh Bm PB1 (SEQ ID NO 2), Mdh1 Bm PB1 (SEQ ID NO 3), Mdh Bm C1 (SEQ ID NO 4), Adh1 Bm (SEQ ID NO 5), Adh2 Bm (SEQ ID NO 6).

Two conserved regions in the Mdh/Mdh1 class enzymes which are less than 80% identical to the homologous regions of Mdh2/Mdh3 class enzymes were identified as being close to the active site, which was identified based on structural comparisons to known alcohol dehydrogenases. The two regions are considered to be relevant for the catalytic activity of the Mdh enzymes. It is assumed that at least one, or both of these regions define the ability, or lack thereof, of the Mdh enzymes to overoxidize methanol to formate.

These conserved regions are:
- Region 1, corresponding to residues 184 to 205 of Mdh Bm MGA 3 (SEQ ID NO 1): AGLTIATGMDALSHAIEAYVAK (SEQ ID NO 13), and
- Region 2, corresponding to residues 248 to 284 of Mdh Bm MGA 3 (SEQ ID NO 1): MAGVAFNNGGLGLVHSISHQVGGVYKLQHGICNSVNM (SEQ ID NO 14).

In one embodiment the Mdh enzyme used in the recombinant methylotrophic strain comprises a sequence having at least 70%, 80%, 90%, 95%, 98% or 99% sequence identity to SEQ ID NO 13 and/or comprising a sequence having at least 70%, 80%, 90%, 95%, 98% or 99% sequence identity to SEQ ID NO 14, wherein said sequences, if both present in the polypeptide are spaced at least 40 residues apart, for example 40 to 50 or 40 to 45 residues apart.

In one embodiment the Mdh polypeptide comprises an N-terminal part having at least 70%, 75%, 80%, 85%, 90%, 95%, 98% or 99% sequence identity to the N-terminal part containing amino acid residues 1-178 of Mdh Bm MGA3 (SEQ ID NO 1).

It was found that the N-terminal part of the Mdh enzyme is relevant for its ability, or lack thereof, to overoxidize methanol into formate.

It was further found that co-expression of a Nudix hydrolase Act (SEQ ID NO 12) improved the oxidation of methanol catalyzed by a Mdh/Mdh1 class enzyme but had no effect on the enzymes inability to further oxidize formaldehyde to formate.

In one embodiment, the recombinant methylotrophic microorganism of this invention therefore also expresses or over-expresses a polypeptide having a Nudix hydrolase Act (SEQ ID NO 12) activity.

In one embodiment the recombinant methylotrophic microorganism of this invention expresses or over expresses all enzymes required for a complete ribulose monophosphate (RuMP) cycle. The microorganism thus expresses or over expresses a polypeptide having methanol dehydrogenase activity, such as Mdh, a polypeptide having 3-hexulose 6-phosphate synthase, such as Hps, a polypeptide having 6-phospho 3-hexuloisomerase activity, such as Phi, a polypeptide having glucose-6-phosphate isomerase activity, such as Pgi, a polypeptide having transketolase activity, such as TktA, a polypeptide having ribulose-phosphate 3-epimerase, such as Rpe, a polypeptide having ribose-5-phosphate isomerase activity, such as RpiA, a polypeptide having transaldolase activity, such as TalA, a polypeptide having NADP⁺-dependent glucose-6-phosphate dehydrogenase, such as Zwf, a polypeptide having 2-keto-3-deoxygluconate 6-phosphate/2-keto-4-hydroxyglutarate aldolase activity, such as Eda, and a polypeptide having a phosphogluconate dehydratase, such as Edd.

The recombinant non-naturally occurring methylotroph may be obtained by genetically modifying a non-naturally occurring methylotrophic bacterial organism selected from a group consisting of *Escherichia,* such as *E. coli, Bacillus,* such as B. *subtilis, Clostridium, Enterobacter, Klebsiella, Mannheimia, Pseudomonas,* such as P. *putida, Acinetobacter, Shewanella, Paracoccus, Ralstonia,* such as *R. eutropha, Geobacter, Zymomonas, Acetobacter, Geobacillus, Lactococcus, Streptococcus, Lactobacillus, Corynebacterium,* such as *C. glutamicum, Streptomyces, Proprionibacterium, Synechocystis, Synechococcus, Cyanobacteria, Chlorobi,* and *Deinococcus.*

In one embodiment the recombinant microorganism of this invention is a methanol-dependent strain. It may for example be a methanol-dependent *E. coli* strain, such as MDep_Ecoli having CCOS accession number CCOS 2133, or an *E. coli* strain having a doubling time and product profile of this strain.

In an alternative embodiment the recombinant microorganism of this invention is a fully methylotrophic strain. It may for example be a fully methylotrophic *E. coli* strain, such as *E. coli* MEcoli_ref_1 having CCOS accession number CCOS 2032, the designated *E. coli* MEcoli_ref 2 having CCOS accession number CCOS 2080, the designated *E. coli* strain SM1 having ATCC accession number PTA-126783, or an *E. coli* strain having a doubling time and product profile of CCOS deposit accession number CCOS 2032, of CCOS deposit accession number CCOS 2080, or of ATCC accession number PTA-126783.

The invention also concerns a recombinant polypeptide having Mdh activity, comprising an N-terminal part with at least 70%, 75%, 80%, 85%, 90%, 95%, 98% or 99% sequence identity to the N-terminal comprising amino acid residues 1-178 of Mdh Bm MGA3 (SEQ ID NO 1).
According to this invention a recombinant polypeptide having Mdh activity may comprise a sequence having at least 70%, 80%, 90%, 95%, 98% or 99% sequence identity to SEQ ID NO 13 and/or comprising a sequence having at least 70%, 80%, 90%, 95%, 98% or 99% sequence identity to SEQ ID NO 14, wherein said sequences, if both present in the polypeptide are spaced at least 40 residues apart, for example 40 to 50 or 40 to 45 residues apart. One or both of said sequences may be comprised in the C-terminal part of the Mdh polypeptide, corresponding to amino acid residues 181 - 382 of Mdh Bm MGA3 (SEQ ID NO 1).

The invention also concerns an expression vector or a cell comprising nucleotide sequences encoding for these recombinant Mdh polypeptides.

The invention further concerns a method for producing or for increasing production of a microbial metabolite or product. For this purpose, the recombinant non-naturally occurring methylotroph microorganism of this invention is grown on a C1 compound, for example methanol. The microorganism converts the C1 compounds into microbial metabolites or products which are subsequently retrieved from the culture.

Depending on the genotype of the strain and the growth conditions, different metabolites or products may be obtained.

Such metabolites or products may for example be one or more of 2-carbon compounds; 3-carbon compounds, such as lactate, or lactic acid; 4-carbon compounds, such as isobutanol, butanol, or 1,4-butanediol; 5-carbon compounds, such as n-pentane, glutamate; higher carboxylic acids, alcohols of higher carboxylic acids; polyhydroxyalkanoates, such as polyhydroxybutyrate (PHB); taxadiene; itaconate; speciality chemicals; aromatic compounds; vitamins; derivatives or precursors of vitamines; carotenoids; amino acids; and sugars, such as deoxyribose

### Definitions

A "recombinant" microorganism refers to a microorganism which has been genetically modified to express or over-express endogenous nucleotide sequences, or to express non-endogenous nucleotide sequences, such as those included in a plasmid, or which has a reduction in expression of an endogenous gene. In analogy, a "recombinant" polypeptide is a polypeptide created through a genetically modified nucleotide sequence of a gene.

A "synthetic" microorganism refers to a recombinant microorganism in which a portion of the genome or the entire genome has been genetically modified.

A "non-naturally occurring methylotrophic microorganism" refers to a microorganism derived from an ancestral microorganism which lacks the ability of methylotrophic growth, but through recombinant engineering or recombinant engineering and laboratory evolution has been adapted to methylotrophic growth.

The term "recombinant methylotroph" as used herein refers to a non-naturally occurring genetically modified methylotroph.

A "methylotrophic" microorganism, also referred to as "methylotroph", is a microorganism which converts C1 compounds, in particular methanol, into biomass. A methylotrophic microorganism may be a "methanol-dependent" microorganism or a "fully methylotrophic" microorganism.

The term "methanol-dependent" microorganism refers to a microorganism which requires methanol as well as other multi-carbon substrates for growth. Even though this microorganism requires methanol for growth, it is unable to grow on C1 compounds as sole carbon source. It requires methanol to grow on certain multi-carbon sources such as pyruvate in the absence of methanol. "Methanol-dependent" therefore indicates the requirement for methanol and at least one multi-carbon compound for growth.

The term "fully methylotrophic" microorganism is a microorganism which uses exclusively reduced C1 compounds as carbon source. Preferably, the main C1 carbon source for a methylotroph is methanol. "Fully methylotroph" or "full methylotrophy" therefore indicate that only C1-compounds, in particular methanol, are required as carbon substrates for growth.

A "chimeric polypeptide" is a polypeptide created through the joining of two or more genes which are originally coded for separate or same proteins.

The singular "a", "an" and "the" include the plural equivalents unless the context distinctly indicates otherwise.

The terms "comprises" and "contains" denote "includes" in a non-limiting sense, while the term "consisting" indicates a limiting sense.

The terms "e.g.", "for example" and "such as" as used herein denote specific non-limiting examples that fall under a more general category.

### Short description of the drawings

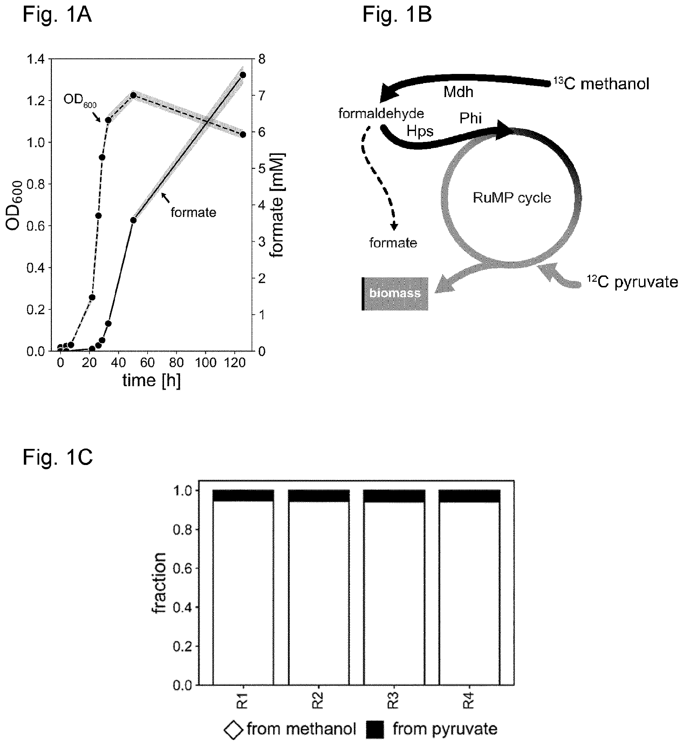

Exemplar embodiments of the invention are disclosed in the description and illustrated by the drawings in which
**Figure 1A** shows a growth curve (Optical density (OD600)) and formate production of the methanol-dependent strain *ΔfrmAΔtpiA* expressing *mdh, hps,* and *phi* (strain MDep_Ecoli) during growth on methanol (500 mM) and pyruvate (20 mM) in shake flasks (n = 3), with shaded region representing the range of the 95% confidence interval;
**Figure 1B** depicts a simplified metabolic scheme illustrating methanol-dependent growth of MDep_Ecoli when cultivated on methanol and pyruvate, with molecules originating from formaldehyde or close to the one-carbon entry point being expected to be formed from methanol (black section of cycle) while others being formed from pyruvate (gray section of cycle), and the canonical formaldehyde detoxification pathway of E. coli, which is inactivated in MDep_Ecoli due to it*s frmA* deletion, being indicated by the dashed line;
**Figure 1C** is a bar chart showing fractions of formate molecules determined in the supernatant of the MDep_Ecoli originating from ¹³C methanol (white) and ¹²C pyruvate (black) when sampled at stationary phase, with R1 to R4 indicating replicate experiments;

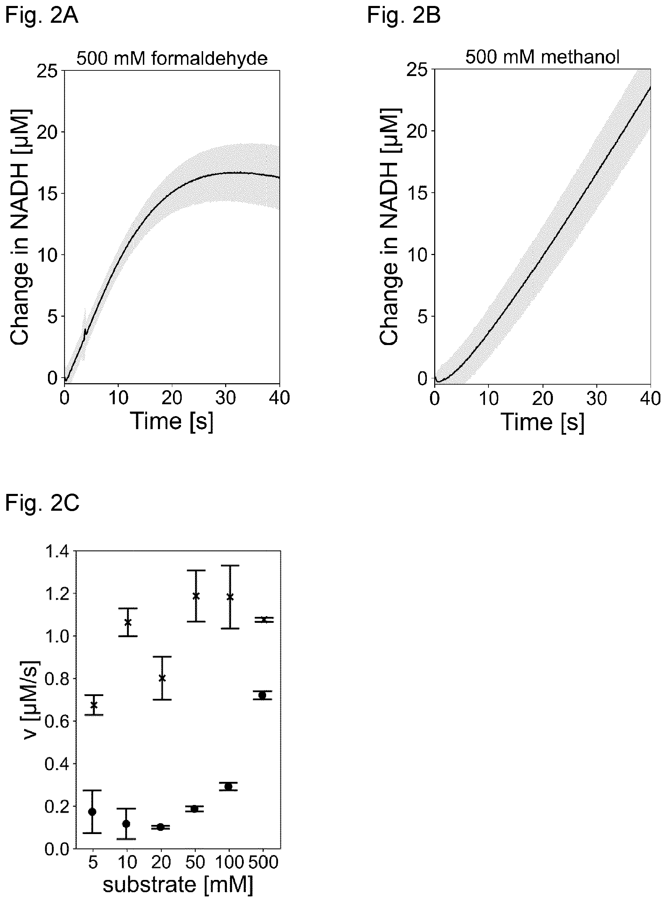

**Figure 2A to 2C** show results from *in vitro* assays of Mdh2 CT4-1 Cn showing the change in NADH concentration by monitoring the absorption at 340 nm, with reaction mixtures containing 12.3 µM enzyme, 5 mM NAD⁺ and reactions being started by the addition of 500 mM formaldehyde **(****Figure 2A****)** or methanol **(****Figure 2B****),** with shaded regions representing the range of 90% confidence interval; and **Figure 2C** summarizes Vₘₐₓ values determined by *in vitro* assays for 10, 20, 50, 100 and 500 mM methanol or formaldehyde as initial concentrations, with methanol to formaldehyde reactions are indicated as dots, formaldehyde to formate indicated as crosses, and error bars representing the standard deviation (n = 4 for 500 mM formaldehyde, n = 3 for remaining conditions);

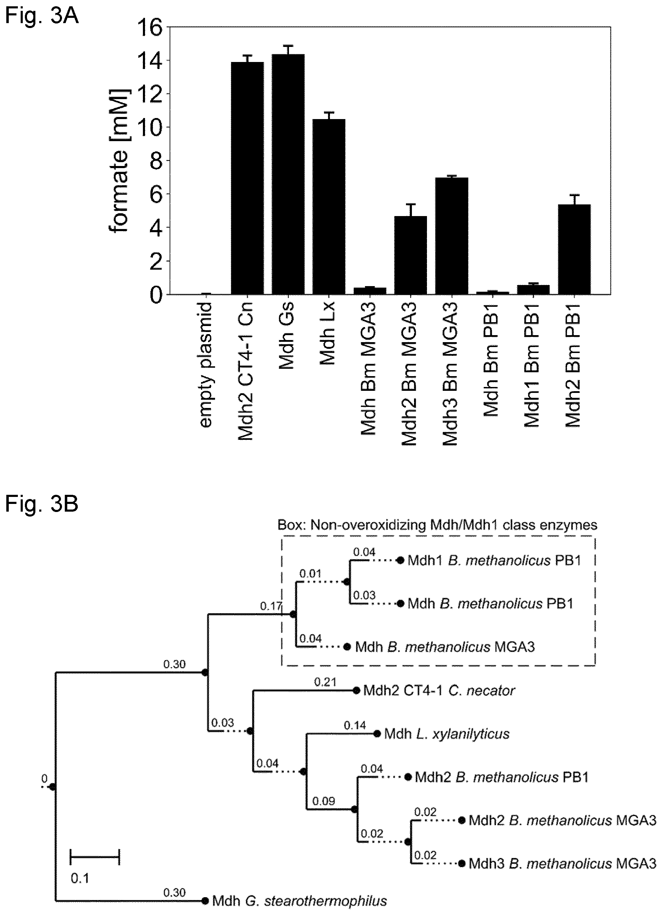

**Figure 3A** shows formate production by different NAD-dependent methanol dehydrogenases, with strains overproducing Mdh in *E. coli* Screen_Ecoli *(ΔfrmRAB)* being grown on methanol (500 mM) and pyruvate (20 mM) and formate being quantified in the supernatant of the cultures after 69 h, whereby the formate levels of a strain harboring an empty plasmid were used as background control and subtracted from each measured value, and error bars represent the standard deviation (n = 3);
**Figure 3B** shows a phylogenetic clustering presentation based on multiple sequence alignment and phylogenetic analysis of Mdh enzymes, which revealed that Mdh enzymes which overoxidize methanol to produce formate are phylogenetically distinct from Mdh enzymes which do not overoxidize methanol and are found in the Mdh/Mdh1 class shown by the box;

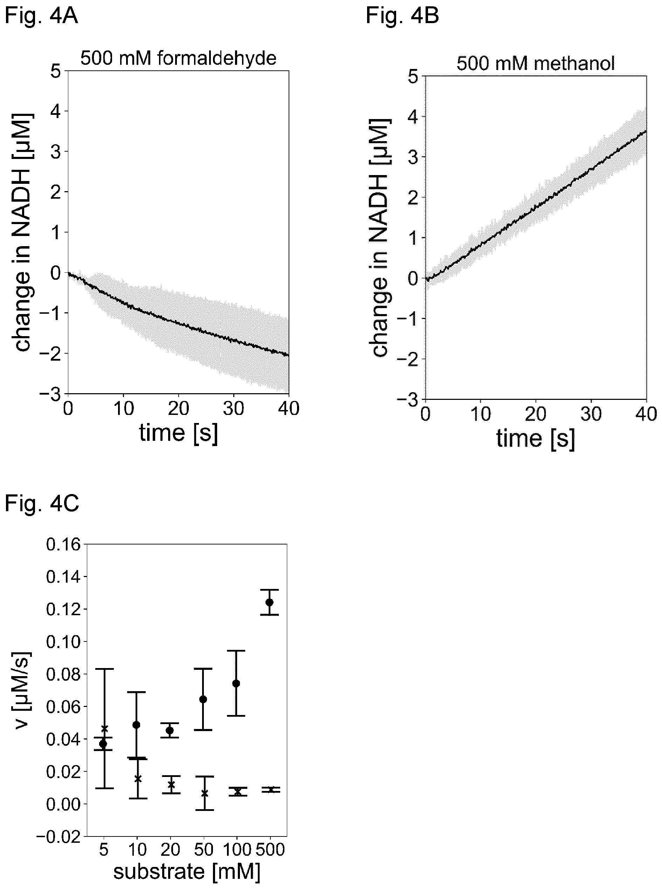

**Figures 4A and 4B** show *in vitro* assays of Mdh Bm MGA3 activity with changes in NADH concentration (absorption at 340 nm) as read-out for enzymatic activity, with reaction mixtures containing 12.5 µM Mdh Bm MGA3, 5 mM NAD⁺ and reactions being started by adding 500 mM formaldehyde **(****Figure 4A****)** or methanol **(****Figure 4B****),** respectively, wherein shaded regions represent the range of the 90% confidence interval;
**Figure 4C** presents a summary of Vₘₐₓ determined by *in vitro* assays for 5, 10, 20, 50, 100 and 500 mM methanol and formaldehyde as initial concentrations, with methanol to formaldehyde reactions are indicated as dots, formaldehyde to formate indicated as crosses, with error bars representing the standard deviation (n=3);

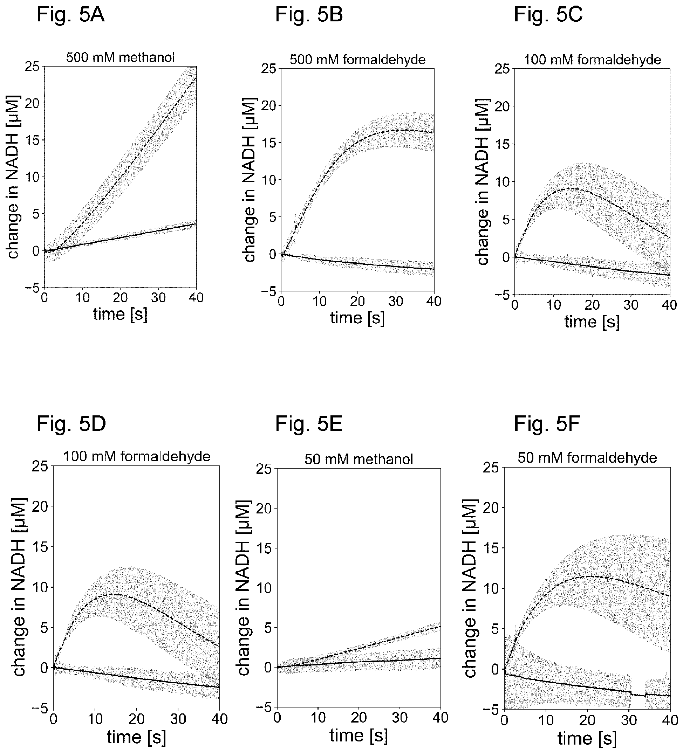

**Figures 5A to 5L** show *in vitro* assays of Mdh activity with changes in NADH concentration (absorption at 340 nm) as read-out for enzymatic activity, with Mdh Bm MGA3 activity indicated by a solid line and overoxidizing Mdh2 CT4-1 Cn activity indicated by an dashed line, with reaction mixtures containing 12.3 (Mdh2 CT4-1 Cn) or 12.5 µM (Mdh Bm MGA3) of the respective enzyme, 5 mM NAD⁺ and reactions being started by adding 5, 10, 20, 50, 100, or 500 mM methanol or formaldehyde, as indicated, with shaded regions representing the range of the 90% confidence interval; all experiments were performed in triplicates;

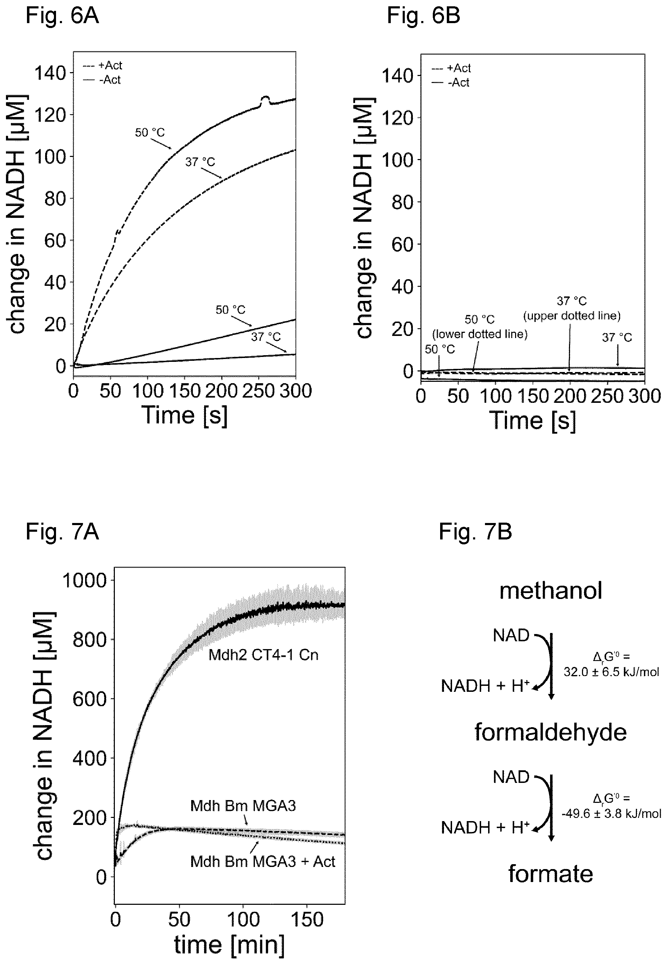

**Figures 6A and 6B** show the results of *in vitro* assays of Mdh Bm MGA3 activity with changes in NADH concentration (absorption at 340 nm) as read-out for enzymatic activity at 37°C and 50°C in the presence (dashed line) or absence (solid line) of the activator protein Act Bm, with **Figure 6A** showing Mdh Bm MGA3 activity with methanol as a substrate, and **Figure 6B** showing Mdh Bm MGA3 activity with formaldehyde as a substrate, with reaction mixtures containing 3.1 µM Mdh, 2.3 µM Act, 5 mM NAD⁺ and being started by adding 500 mM methanol or 2 mM formaldehyde, respectively;
**Figure 7A** shows changes In NADH levels of *in vitro* assays of Mdh2 CT4-1 Cn (solid line), Mdh Bm MGA3 (dashed line), and Mdh Bm MGA3 with Act (dotted line) monitored until equilibrium was reached, with reaction mixtures containing 25 µM Mdh2 CT4-1 Cn or 25 µM Mdh Bm MGA3, 2.3 µM Act if indicated, 5 mM NAD⁺ and reactions being started by adding 500 mM methanol;
**Figures 7B** shows the two oxidation steps catalysed by Mdh enzymes of the Mdh2 group, with a first oxidation step from methanol to formaldehyde and a second oxidation step from formaldehyde to formate; ΔrG'0 values were calculated by eQuilibrator (pH = 7.4, pMg = 2.3, ionic strength = 0.15 M);

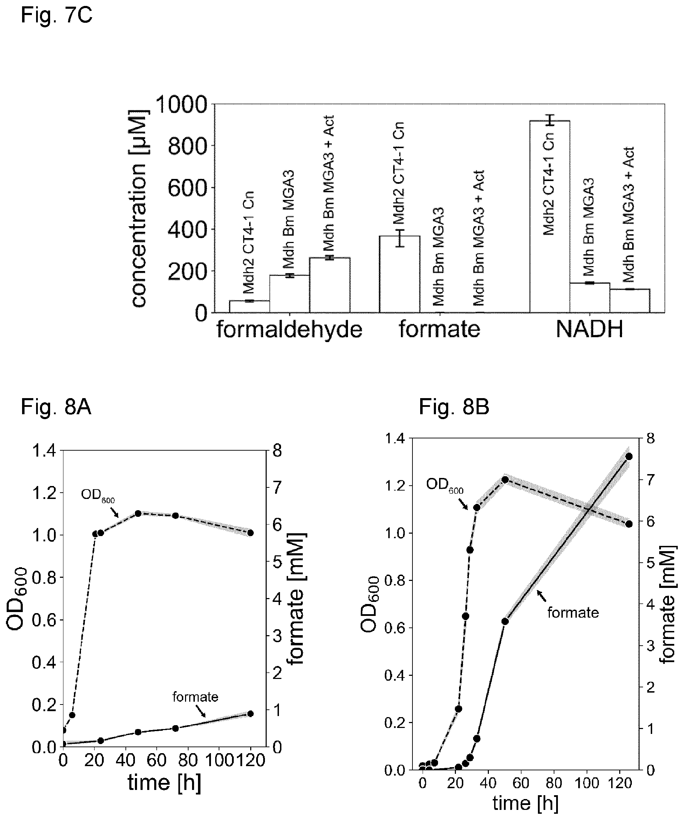

**Figure 7C** shows equilibrium concentrations of formaldehyde, formate, and NADH with reaction mixtures containing 1 mg mL⁻¹ of the respective enzyme, 5 mM NAD⁺ and reactions being started by adding 500 mM methanol; all experiments were performed in triplicates;
**Figures 8A and 8B** show growth curves (optical density at 600nm) (dashed line) and formate production (solid line) of methanol-dependent strain Screen_Ecoli *(ΔfrmAΔtpiA)* expressing the non-overoxidizing *mdh* Bm MGA3 (MDep_Ecoli) **(****Figure 8A****)** or *mdh2* CT4-1 Cn **(****Figure 8B****)** during growth on methanol (500 mM) and pyruvate (20 mM) (n = 3), with shaded region representing the range of the 95% confidence interval;

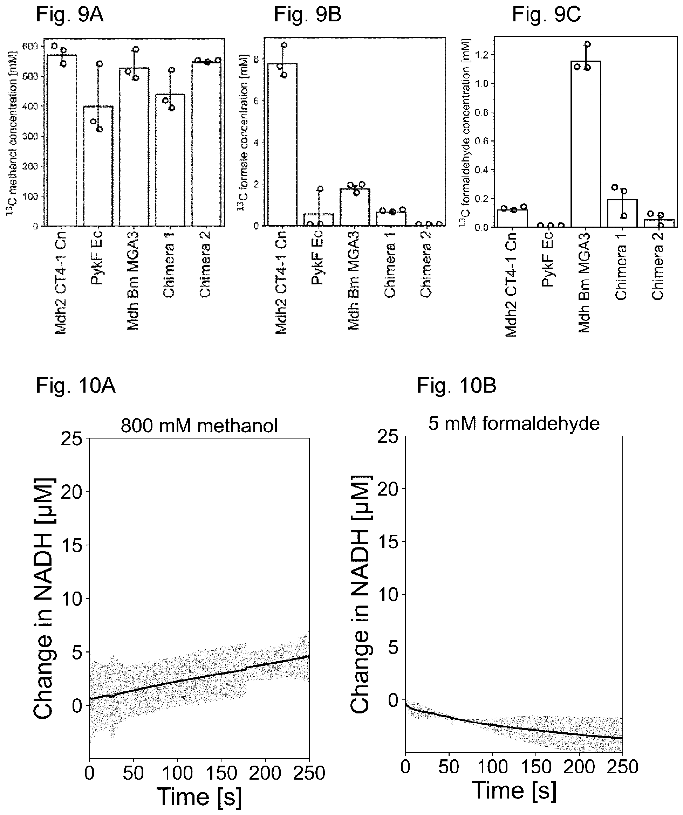

**Figures 9A****, B, C** show the methanol (A), formaldehyde (B) and formate (C) concentration in the supernatant of whole cell assays on BL21 *ΔfrmA* strains, each expressing a different methanol dehydrogenase enzyme (Mdh2 CT4-1 Cn (overoxidizing control), Mdh Bm MGA3 (non-overoxidizing control), Chimera 1 (N-terminal part of Mdh Bm MGA3 (amino acids 1- 178) linked with MV to C-terminal part of Mdh2 CT4-1 Cn (amino acids 189-390), Chimera 2 (N-terminal part of Mdh2 CT4-1 Cn (amino acids 1-186) linked with MV to C-terminal part of Mdh Bm MGA3 (amino acids 181-382). Pyruvate kinase 1 (PykF) was used as negative control. Whole cell assays were started with 800 mM ¹³C methanol and metabolites were quantified in the supernatant after 10 h. Error bars represent the standard deviation of three replicates.
**Figures 10A and 10B** show *in vitro* assays of Chimera 1 (N-terminal site Mdh Bm MGA3 and C-terminal site Mdh2 CT4-1 Cn) activity with changes in NADH concentration (absorption at 340 nm) as read-out with reaction mixtures containing 126.5 µM enzyme, 5 mM NAD⁺ and reactions being started by adding 800 mM methanol **(****Figure 10A****)** or 5 mM formaldehyde **(****Figure 10B****),** as indicated, with shaded regions representing the range of the 90% confidence interval; experiment started from methanol were performed in duplicates and from formaldehyde in triplicates;

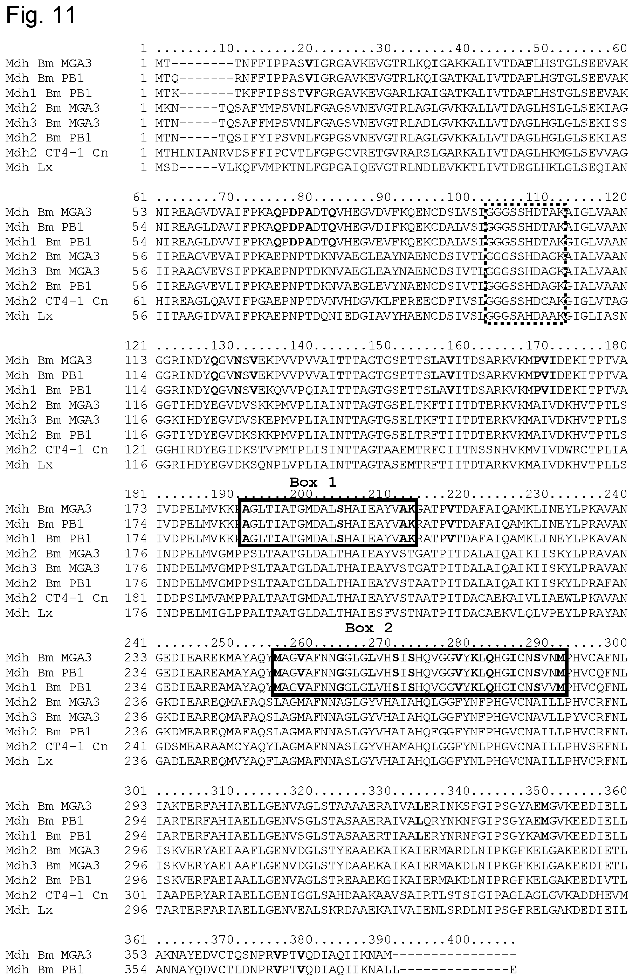

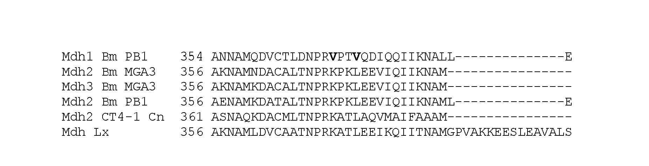

**Figure 11** depicts a sequence alignment of non-overoxidizing Mdh enzymes (Mdh Bm MGA3 (SEQ ID NO 1), Mdh Bm PB1 (SEQ ID NO 2), Mdh1 Bm PB1 (SEQ ID NO 3)), and overoxidizing Mdh enzymes (Mdh2 Bm MGA3 (SEQ ID NO 7), Mdh3 Bm MGA3 (SEQ ID NO 8), Mdh2 Bm PB1 (SEQ ID NO 9), Mdh2 CT4-1 Cn (SEQ ID NO 10), Mdh Lx (SEQ ID NO 11)), with the dashed box indicating the NAD-binding site, and the solid line boxes 1 and 2 indicating regions which are presumed to be relevant in defining overoxidation capabilities of the enzymes.

### Examples of embodiments of the present invention

The genes *mdh* Bm MGA3 and *mdh* Bm PB1 are essential for methylotrophy in their native hosts *Bacillus methanolicus.* They are expressed from a multi-copy endogenous plasmid and the encoded Mdh enzymes constitute up to 22% of the proteome in the natural methylotrophic *B. methanolicus* strains MGA3 and PB1.

The gene *mdh1* Bm PB1 also belongs to the Mdh/Mdh1 class but is expressed from the genome. The genomes of *B. methanolicus* strains MGA3 and PB1 also contain genes encoding Mdh enzymes that were previously categorized as Mdh2/Mdh3 type enzymes.

Even though it was known previously that Mdh enzymes can phylogenetically be categorised in two distinct classes or clusters, the significance of this classes was previously not recognized. The separation of Mdhs into two classes was based on their respective amino acid sequences, which are at least 30% different. Similar *in vitro* activity on methanol had previously been reported for the Mdh/Mdh1 and Mdh2/Mdh3 type enzymes and the physiological relevance of their difference remained unclear. In other words, no particular phenotypic feature distinguishing between the two classes had been identified.

### Synthetically methylotrophic E. coli accumulate formate as a side-product during growth

Formate accumulation was for example observed by the previously reported recombinant fully methylotrophic E. coli strain MEcoli_ref_2 (WO2024018304Al) when grown on methanol. When grown in a bioreactor on methanol, formate was produced in the mM range (^{~} 3 mM, 200 mg L⁻¹). Even higher concentrations of formate, up to 38 mM, accumulated during the production of itaconic acid or lactic acid from methanol. In each of the production batches, formate was produced in excess of the intended product (2-fold excess in molar concentration for lactic acid production and up to 37-fold excess in molar concentration for itaconic acid production)

To examine whether formate production was a result of the adaptive laboratory evolution (ALE) over approximately 1200 generations this fully-methylotrophic *E. coli* strain MEcoli_ref_2 had been subjected to, formate production of its ancestral strain , which is a methanol-dependent strain (*ΔfrmAΔtpiA*) heterologously expressing *mdh, hps,* and *phi,* was tested in the same experimental setup. The ancestral strain also produced formate (^{~} 8 mM) (Figure 1A), indicating that formate generation is intrinsic to the strain rather than the result of ALE toward fully methylotrophic growth. Synthetic constructs were used for heterologous expression of Mdh Bm MGA3 (pSEVA434_mdh_Bm_MGA3, Seq ID NO 15) and Hps and Phi proteins (pSEVA131_hpi_phi_Mf, Seq ID NO 16).

The metabolic origin of formate production in absence of *frmA,* which encodes a key enzyme of the formaldehyde detoxification pathway, formaldehyde-dependent S-(hydroxymethyl)glutathione dehydrogenase, was however not well understood.

One potential mechanism for formate generation could be the activity of an alternative dehydrogenase that compensates for the lack of *frmA* in the strain. To investigate this, the entire formaldehyde detoxification pathway operon *(ΔfrmRAB)* was deleted in the ancestral strain , resulting in strain . However, the deletion of the entire operon did not result in a reduction of formate production which points to a different route for formate production by the methylotrophic E. coli strain.

It was speculated that formate originates from a metabolite close to the one-carbon entry point into the RuMP cycle. To test this hypothesis the ancestral methanol-dependent strain was grown under defined conditions in which overall carbon flux from methanol and pyruvate is well characterized and consistent with flux balance analysis. By feeding ¹³C labelled methanol and unlabelled pyruvate, the origin of formate was traced on the basis of its labelling pattern (Figure 1B). The results revealed that more than 95% of the formate originated from methanol (Figure 1C), indicating that the formate produced by the strain derives from either formaldehyde or a RuMP cycle intermediate close to the one-carbon entry point.

Since the methanol dehydrogenase (Mdh) is one of the three heterologously expressed enzymes in recombinant methylotrophic strains, it was a possible candidate for driving the production of formate, possibly through direct oxidation of formaldehyde, which is the product of the Mdh catalysed oxidation of methanol, to formate.

It was therefore tested whether this enzyme could catalyse both reactions, i.e. the oxidation from methanol to formaldehyde, as well as the oxidation of the formaldehyde to the obtained formate product, thereby preventing formaldehyde from being metabolized through the RuMP cycle. In aqueous solution, formaldehyde exists predominantly as methylene glycol. It was speculated methylene glycol could possibly be recognized as a substrate by Mdh.

### NAD-dependent methanol dehydrogenase Mdh2 CT4-1 Cn oxidizes formaldehyde to formate in vitro

To test this hypothesis, the Mdh enzyme Mdh2 CT4-1 Cn (SEQ ID NO 10), which is the heterologously introduced methanol dehydrogenase in the methanol-dependent ancestral strain and its descendant methylotrophic strains MEcoli_ref_1 and MEcoli_ref_2 (WO2024018304A1). The Mdh2 CT4-1 variant used in this study (SEQ ID NO 10) contains point mutations A26V, A31V, A169V compared to the wild-type Mdh2 CT4-1 enzyme.

To investigate whether Mdh2 CT4-1 Cn oxidizes formaldehyde to formate, the enzyme was purified from an E. coli BL21 overexpression culture and characterized in *vitro.* NADH formation was monitored upon addition of 500 mM formaldehyde to a reaction mixture containing 12.3 µM Mdh and 5 mM NAD⁺, and buffer at 37°C.

NAD⁺ reduction to NADH was observed immediately after the addition of formaldehyde (Figure 2A), confirming that the enzyme catalyzes the oxidation of formaldehyde to formate. As expected, NADH formation was also observed when the reaction was started from methanol (Figure 2B). Further experiments at varying substrate concentrations revealed that formaldehyde oxidation proceeds with substantially higher turnover rates than methanol oxidation (Figure 2C).

These findings demonstrate that Mdh2 CT4-1 Cn overoxidizes methanol to formate and suggest that Mdh is responsible for formate production by synthetically methylotrophic *E. coli* strains.

### Overoxidation of methanol by NAD-dependent methanol dehydrogenases is common

To examine whether overoxidation is a general feature of NAD-dependent Mdh, nine different NAD-dependent Mdhs were overexpressed in an E. coli strain Screen_Ecoli lacking the canonical formaldehyde detoxification pathway (*ΔfrmRAB*)*.* When grown on methanol and pyruvate, six of the tested Mdh enzymes produced between 4 mM and 14.5 mM formate, whereas the three other enzymes others produced only minor levels of less than 0.5 mM of formate (Figure 3A).

The Mdh enzymes producing formate from methanol by a two-step oxidization of methanol to formaldehyde and subsequently of formaldehyde to formate, are herein referred to as overoxidizing enzymes. In contrast, the Mdh enzymes which are catalysing the first oxidation step, i.e. the oxidation of methanol to formaldehyde, only, are herein referred to as non-overoxidizing enzymes.

Multiple sequence alignment and phylogenetic analysis revealed that the non-overoxidizing enzymes form a separate class, sharing less than 70% sequence identity with the remaining enzymes (Figure 3B). This class has previously been described as the Mdh/Mdh1 in *B. methanolicus* strains (Krog et al; PLoS ONE 8, (2013), DOI : 10.1371/journal.pone.0059188), where these enzymes represent the predominant methanol dehydrogenase in these organisms. The physiological relevance of the enzyme class had however previously not been identified.

The results obtained in this study revealed a distinct catalytic activity of the Mdh/Mdh1 class compared to other bacterial methanol dehydrogenases. This is the first evidence for a potential difference in function of NAD-dependent methanol dehydrogenases in methanol physiological pathways. The observed non-overoxidation of methanol of the Mdh/Mdh1 class, in contrast to other Mdh enzymes, may be the key characteristic of this class of Mdh enzymes.

Based on these results it is postulated that NAD-dependent methanol dehydrogenases (Mdh) fall in two groups, which are a first group of overoxidizing enzymes, converting methanol to formate, and a second group of non-overoxidizing enzymes, oxidizing methanol to formaldehyde, but not catalysing the second oxidation step of formaldehyde to formate. Mdh enzymes of this second group form part of the phylogenetic Mdh/Mdh1 class.

### Mdh Bm MGA3 does not oxidize formaldehyde in vitro

The *in vivo* Mdh screen resulted in the identification of three candidate methanol dehydrogenases that do not overoxidize methanol to formate *in vivo.* To further validate this property, one of these three enzymes, Mdh Bm MGA3 (SEQ ID NO 1), was purified from an *E. coli* BL21 overexpression culture for *in vitro* assays.

In contrast to Mdh2 CT4-1 C, which does not form part of the Mdh/Mdh1 phylogenetic cluster of Mdh enzymes, no NADH was detected in the *in vitro* Mdh activity assay using Mdh Bm MGA3 when formaldehyde (500 mM) was used as a substrate. This result demonstrates that Mdh Bm MGA3, which is one of the Mdh/Mdh1 enzymes, does not oxidize formaldehyde (Figure 4A), confirming the in vivo results for this enzyme.

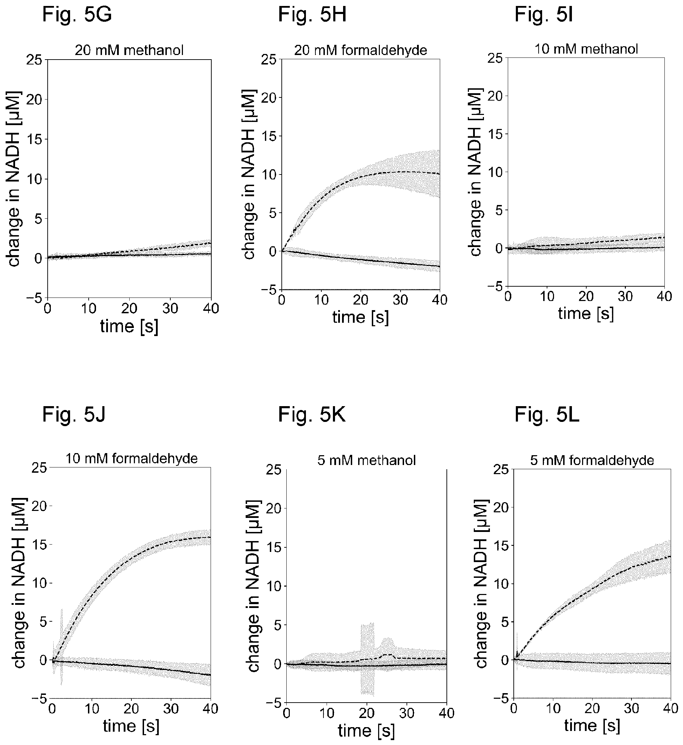

When starting the reaction with the same concentration of methanol, NADH formation was observed (Figure 4B). In comparison to Mdh2 CT4-1 Cn, the turnover rate for the oxidation of methanol by Mdh Bm MGA3 was about 7 times slower, which is in line with previous reports due to the inferior catalytic efficiency and difference in temperature optimum of Mdh Bm MGA3. To avoid a potential inhibitory or inactivating effect of a high concentration formaldehyde, which had been provided at 500mM, lower initial formaldehyde concentrations were also tested. Interestingly, over a range of 5 mM to 500 mM of formaldehyde, Mdh MGA3 showed no observable oxidation activity (Figure 4C, Figures 5B, D, F, H, J, and L, solid lines). In contrast, the Mdh2/Mdh3 type enzyme Mdh2 CT4-1 Cn showed rapid oxidization of formaldehyde over the whole range of concentrations (Figures 5B, D, F, H, J, and L, dashed lines).

It is also noteworthy, that Mdh2 CT4-1 has a higher turnover rate than Mdh Bm MGA3 when methanol is provided as a substrate, as shown in Figures 5A,C,E,G,I and K), in particular at concentrations of 50mM and above.

### Mdh Bm MGA3 does not oxidize formaldehyde in vitro in the presence of a Nudix hydrolase

NAD-dependent methanol dehydrogenases are known to be activated by Nudix hydrolases, resulting in superior catalytic efficiency *in vitro.* To investigate whether the activation activity of the Nudix hydrolase Act of *B. methanolicus* could in any way serve to overcome the lack of ability of Mdh Bm MGA3 to oxidize formaldehyde, Act was purified from a BL21 *E. coli* overexpression culture and the formaldehyde oxidization assay using Mdh Bm MGA3 was repeated the *in vitro* assay in the presence of Nudix hydrolase Act.

As shown in Figure 6B, no NADH was generated when the reaction was started with formaldehyde in the presence of Act, regardless of whether the assay was performed both at the optimal growth temperature of *E. coli* (37°C) or that of B. *methanolicus* (50°C), indicating that no oxidation of formaldehyde in the assay occurred. In contrast, when methanol was provided as a substrate for Mdh Bm MGA3, the Nudix hydrolase Act greatly increased oxidation activity of the Mdh enzyme regardless of whether the reaction was performed at 37°C or at 50°C (Figure 6A).

### Thermodynamic equilibrium analyses validate non-overoxidizing Mdh

To learn more about the respective enzymatic activities of overoxidizing enzymes, for example Mdh2/Mdh3 type enzymes, compared to non-overoxidizing Mdh/Mdh1 type enzymes, the equilibrium states of the *in vitro* reactions for Mdh2 CT4-1 Cn, an overoxidizing enzyme, and Mdh Bm MGA3, a Mdh/Mdh1 type enzyme, were compared. In these experiments, 500 mM methanol, 5 mM NAD⁺, and 25 µM Mdh were incubated until equilibrium was reached. The enzymatic activity of Mdh Bm MGA3 was also tested in the presence of Act.

The time to reach equilibrium varied substantially between the different reactions, with Mdh Bm MGA3 in the presence of Act requiring the shortest time due to the higher catalytic activity (Figure 7A).

In addition, it was found that metabolite concentrations at equilibrium differ between overoxidizing Mdh2 CT4-1 Cn and non-overoxidizing Mdh Bm MGA3. Consistent with the hypothesis of one versus two-step oxidation process (methanol to formaldehyde, and formaldehyde to formate), the final NADH levels differed markedly between the overoxidizing and non-overoxidizing Mdh enzymes (900 µM versus 140 µM formed NADH), irrespective of Act addition.

Thermodynamic calculations based on the Gibbs free energy under standard conditions for methanol oxidation to formaldehyde (ΔGr'0 = 32.0 ± 6.5 kJ/mol) and formaldehyde oxidation to formate (ΔGr'0 of -49.6 ± 3.8 kJ/mol) (Figure 7B) predicted roughly 100 µM for the non-overoxidizing reaction under the experimental conditions, closely matching the observed value (Figure 7C).

For an overoxidizing Mdh, the calculated NADH concentration was close to 5 mM which is higher than what was measured (920 µM). Similarly, measured formaldehyde and formate concentrations at equilibrium deviated from theoretical values in the overoxidizing reaction (57 µM vs 0.01 µM formaldehyde and 368 µM vs 2.5 mM formate), suggesting constraints such as limited affinity for formaldehyde or incomplete equilibration within the experimental timeframe. These results further support the initial findings regarding the inability of Mdh Bm MGA3 to oxidize formaldehyde.

### Replacement of overoxidizing with non-overoxidizing methanol dehydrogenase reduces the accumulation of formate during methanol-dependent growth

The *in vivo* and *in vitro* experiments described above showed that Mdh Bm MGA3, unlike overoxidizing Mdh enzymes, does not oxidize formaldehyde. This finding identifies Mdh/Mdh1-type enzymes as promising candidates for previously reported synthetically methylotrophic *E. coli* strains, in which only overoxidizing Mdh enzymes have been introduced and utilized so far.

To address the problem of unwanted production of formate by these synthetic methylotrophs, this invention therefore provides methylotrophic strains expressing Mdh/Mdh1 type enzymes instead of the previously used overoxidizing Mdh enzymes, thereby reducing or preventing the accumulation of formate. As a further important aspect, the use of Mdh/Mdh1 type enzymes in synthetic methylotrophs also serves to maintain a steady pool of formaldehyde for assimilation purposes, since formaldehyde is not further oxidized to formate.

To further confirm the efficacy of the proposed solution, it was tested whether a non-overoxidizing Mdh is sufficient to sustain growth of *E. coli* in the presence of methanol without causing the accumulation of formate.

For this purpose, two of the non-overoxidizing mdh genes (mdh Bm MGA3 and mdh Bm PB1, respectively) in addition to the RuMP cycle genes hps and phi were introduced into the ΔfrmAΔtpiA background to generate two different strains expressing either mdh Bm MGA3 or mdh Bm PB1. Methanol-dependent growth has the advantage that it can be readily established due to the lower biomass contribution of methanol. The two strains were adapted for methanol-dependent growth as previously described for mdh2 CT4-1 Cn (Keller, P. et al.; Nat. Commun. 11, 5403 (2020), DOI: 10.1038/s41467-020-19235-5) to generate two distinct methanol-dependent strains. Strikingly, 8 times lower amounts of formate (Figure 8A) as for the methanol-dependent strain expressing *mdh2* CT4-1 Cn (Figure 8B) was observed for the strain expressing *mdh* Bm MGA3.

These results demonstrate that methanol-dependent growth of *E. coli* using a non-overoxidizing Mdh, in this case Mdh Bm MGA3, is possible and that formate accumulation is drastically reduced in this strain. The replacement of Mdh also indicates that overoxidation of methanol by Mdh2 CT4-1 Cn was responsible for formate accumulation in previously described synthetically methylotrophic *E. coli* strains which expressed this overoxidizing enzyme.

### N-terminal part of Mdh potentially important for overoxidation

To evaluate the regions of the Mdh enzyme which determine the enzyme's ability of oxidize formaldehyde, chimeric Mdh proteins were generated.

A first chimera (Chimera 1, SEQ ID NO 17) contained the C-terminal part (amino acids 189-390) of Mdh2 CT4-1 Cn and the N-terminal part (amino acids 1- 178) of Mdh Bm MGA3 (non-overoxidizing N-terminal part). The two parts were linked with an overlapping sequence of two amino acids (MV) which is present in both enzymes. A second chimera (Chimera 2, SEQ ID NO 18) contained the C-terminal part (amino acids 181-382) of Mdh Bm MGA3 (non-overoxidizing C-terminal part) and the N-terminal part (amino acids 1-186) of Mdh2 CT4-1 Cn. Both parts were linked with an overlapping sequence of two amino acids (MV) which is present in both enzymes.

A whole cell assay was performed on the two chimera proteins, in which the chimeras were overexpressed in a BL21 *ΔfrmA* strain (Figures 9A, B, C). Mdh Bm MGA3 was used as non-methanol-overoxidizing control, Mdh2 CT4-1 Cn as methanol overoxidizing control. Chimera 1 produced low amounts of formaldehyde (Figure 9B) while not producing formate (Figure 9C), indicating that the N-terminal part of Mdh Bm MGA3 is important for the absence of overoxidation. Chimera 2 activity was not detected.

The absence of overoxidation by Chimera 1 was confirmed by purifying it and testing it *in vitro.* As seen in the whole cell assay, activity for the catalysis of the reaction of methanol to formaldehyde was detected (Figure 10A), while no activity was observed for the reaction from formaldehyde to formate (Figure 10B).

A sequence alignment of three non-overoxidizing Mdh enzymes (Mdh Bm MGA3, Mdh Bm PB1, Mdh1 Bm PB1), belonging to the Mdh/Mdh1 class enzymes, and five overoxidizing Mdh enzymes (Mdh2 Bm MGA3, Mdh3 Bm MGA3, Mdh2 Bm PB1, Mdh2 CT4-1 Cn, Mdh Lx), belonging to the Mdh2/3 class enzymes, is shown in Figure 11. The box with a dashed line indicates the position of the NAD-binding site in these enzymes. The solid line boxes 1 and 2 residues which are either part of or in proximity to the active site.

Interestingly, the conserved sequences of boxes 1 and 2 differ considerably between the Mdh/Mdh1 class and the Mdh2/3 class of the Mdh enzmyes. Without wishing to be bound by any particular hypothesis, it is postulated that these conserved regions are crucial for the enzymes ability to oxidize formaldehyde to formate.

In particular, conserved region 1 corresponding to residues 184 to 205 of Mdh Bm MGA3 consists of the conserved sequence SEQ ID NO 13:

### AGLTIATGMDALSHAIEAYVAK

Conserved region 2 corresponding to residues 248 to 284 Mdh Bm MGA3 consists of the conserved sequence SEQ ID NO 14:

### MAGVAFNNGGLGLVHSISHQVGGVYKLQHGICNSVNM

It is further postulated that recombinant Mdh enzymes of the overoxidizing class have reduced or no ability to oxidize formaldehyde to formate, if the corresponding regions of a recombinant overoxidizing class enzymes is replaced by either one or by both of the conserved regions 1 and/or 2 of the Mdh/Mdh1 class enzyme.

In short, the absence or presence of at least one of the two conserved regions 1 (SEQ ID NO 13) and 2 (SEQ ID NO 14) in the Mdh enzyme is thought to be important for the turn-over rate of formaldehyde as a substrate.

In addition to the conserved regions 1 and 2, one or more of selected conserved aa residues, which are identified in bold in Figure 10, may define the ability of the Mdh enzymes to oxidize formaldehyde.

These residues correspond to the following positions in the Mdh Bm MGA3:
V12, 113, I28, F40, Q68, D70, A72, Q75, L91, I94, Q120, N123, V125, V132, V133, T136, L148, V150, P161, V162, I163, A184, I188, S196, A204, K205, V210, M248, V251, G256, L260, S263, S265, V271, K273, Q275, I278, S281, N283, M284, L326, M342, S364 V368, V371.

Of these residues A184, I188, S196, A204, K205 and M248, V251, G256, L260, S263, S265, V271, K273, Q275, I278, S281, N283, M284 are of particular interest, as these residues are contained in the conserved regions 1 and 2.

It is speculated that one or more of these residues may be relevant in defining the ability of the Mdh enzymes to oxidize formaldehyde.

It may thus be possible, to introduce overoxidizing properties in the Mdh Bm MGA3 enzyme, or another Mdh/Mdh1 class enzyme, by replacing one or more of the conserved residues with the corresponding residues in the overoxidizing class enzymes:
V12L, I13F, I28L, F40G, Q68E, D70N, A72T, Q75N, L91I, 194L, Q120E, N123D, V125S, V132L, V133I, T136N, L148F, V150I, P161A, V162I, I163V, A184P, I188A, S196T, A204S, K205T, V210I, M248L, V251M, G256A, L260Y, S263A, S265A, V271F, K273N, Q275P, I278V, S281A, N283L, M284L, L326I, M342L, S364T V368K, V371L

In analogy, it may also be possible to replace these corresponding conserved residues in an overoxidizing class enzyme with the above listed residues of Mdh Bm MGA3, to reduce or abolish overoxidizing activity of the overoxidizing class enzyme.

As part of this study, it was shown for the first time that the overoxidation of methanol to formate is a common characteristic among NAD-dependent methanol dehydrogenases. Even though this ability is common, it is not universal and it was further found that Mdh enzymes belonging to the Mdh/Mdh1 class do not share this ability to catalyze the oxidation of formaldehyde to formate. Instead, these enzymes only catalyze the oxidation of methanol to formaldehyde.

The difference between these two classes of Mdh enzymes is highly relevant, as it permits to generate recombinant methylotrophic strains which metabolise methanol through the RuMP cycle and do not accumulate formate as a result of overoxidization of the methanol substrate. The findings therefore allow for a more efficient industrial production of value-added metabolites without the production of significant amounts of formate as by-product.

Moreover, relevant regions in the Mdh enzmyes were identified which impact the enzymes oxidation ability.

The capability of the overoxidizing class enzymes to oxidize formaldehyde to formate suggests that their main role is not the oxidation of methanol but the detoxification of formaldehyde. Canonically, *B. methanolicus* detoxifies formaldehyde via the oxidative pentose phosphate pathway, which requires the regeneration of the one-carbon acceptor ribulose 5-phosphate (Ru5P). However, when Ru5P regeneration is ineffective, formaldehyde detoxification cannot take place. In this case, the overoxidizing class enzymes might serve as a backup system for the detoxification of formaldehyde, while at the same time supporting the oxidation of methanol to formaldehyde as soon as Ru5P pools have been restored. It might thus be particularly important during transition phases of changing environmental conditions with sudden up- or down shifts of methanol. Whether such a system has only evolved in *B. methanolicus* remains open, but so far, this is the only species in which both overoxidizing and non-overoxidizing Mdh enzymes have been found.

The finding that Mdh/Mdh1 type enzymes catalyze oxidation of methanol to formaldehyde and that overoxidizing enzymes catalyze oxidation methanol to formate, is not only important for our understanding of natural methyltrophs but also to inform the engineering of synthetically methylotrophic microbial strains, for example *E. coli strains,* for product formation.

Production yields close to the theoretical maximum will be important to realize economically profitable bioprocesses from methanol by methylotrophic organisms. The production of formate by synthetically methylotrophic *E. coli* has a negative impact on the process yield. To enable its assimilation, an additional heterologous pathway such as the reductive glycine cycle could be implemented. However, its coordination with the already introduced RuMP cycle would be challenging. Notably, the overoxidation of methanol to formate is not a problem for a strain that exclusively utilizes the reductive glycine cycle.

Another potential solution to utilize the formate would be the expression of a formate dehydrogenase. While the carbon would still be lost, the oxidation of formate to CO₂ would provide additional reducing equivalents for the production of chemicals. However, this would only be beneficial for highly reduced products as methanol as a substrate already provides an excess of reducing equivalents for most industrially relevant products.

As an ideal solution, the production of formate is therefore prevented to avoid any loss of carbon and reducing equivalents.

This aim is attained by the present invention, based on the use of non-overoxidizing Mdh enzmyes in recombinant metholotrophic strains, leading to a significant reduction of formate formation in bioproduction cultures. The generation of a synthetically methylotrophic *E. coli* strain which does not produce formate as side product provides an important step towards economical bioprocesses to produce green chemicals from methanol and the establishment of a circular carbon economy.

### Methods

### Plasmids

In the experiments for Figure 3A and Figure 8A, the RuMP cycle genes were expressed from plasmids based on the pSEVA434 and pSEVA131 backbones from the synthetic methylotrophic *E. coli* strain MEcoli_ref_2 (pSEVA434_mdh_Bm_MGA3, Seq ID NO 15, and pSEVA131_hpi_phi_Mf, Seq ID NO 16).

For Figure 1A and Figure 8B, plasmids were isolated from the evolving population in between MEcoli_ref_1, an earlier methylotrophic isolate, and MEcoli_ref_2 (WO2024018304A1). The coding sequence for methanol dehydrogenase 2 variant CT4-1 from *Cupriavidus necator (mdh2* CT4-1 Cn, SEQ ID NO 10) was synthesized by Eurofins.

Methanol dehydrogenase from Bacillus methanolicus MGA3 (mdh Bm MGA3, SEQ ID NO 1) encoding gene was amplified from pET21a plasmids.

For expression and purification of *mdh2* CT4-1 Cn and *mdh* Bm MGA3, the genes were amplified by PCR using primers with overhangs. The PCR products were cloned into a modified pET-16b expression vector where the His₁₀-tag and linker region had been omitted, using Gibson Assembly.

pET16b plasmid constructs encoding *mdh2* CT4-1 Cn and *mdh* Bm MGA3 were transformed into *E. coli* DH5α, and correct plasmid assembly was confirmed by Sanger sequencing. For heterologous expression, the obtained plasmids were transformed into *E. coli* BL21-Gold (DE3) cells. pET16b vectors were used for expression of enzymes which were then purified for in vitro experiments.

pET21a expression plasmid containing the activator *act* Bm MGA3 (Uniprot No. I3EA59, Arfman et al (J. Biol. Chem. (1991)), DOI: 10.1016/50021-9258(19)67886-5) have been described earlier and were received from SINTEF, Norway. It was transformed into *E. coli* BL21-Gold (DE3) cells for expression.

Plasmids used to screen different NAD-dependent methanol dehydrogenases for overoxidation were cloned using isothermal assembly utilizing the commercially available HIFI DNA Assembly mastermix.

### Strains

The strains used in this study are listed in Supplementary Data 1. The synthetic methylotrophic *E. coli* strain MEcoli_ref_2 is able to grow on methanol as its sole carbon and energy source and was described in a previous study (WO2024018304A1).

The methanol-dependent *E. coli* strain ΔfrmAΔtpiA expressing the RuMP cycle genes *mdh, hps* and *phi,* also referred to as MDep_Ecoli, having the CCOS accession number CCOS 2133, was generated using the previously described ancestral strain for the adaptive laboratory evolution (Keller et al., Nat. Comm. (2020), DOI: 10.1038/s41467-020-19235-5) which generated *E. coli* strain MEcoli_ref_2 (Keller et al (Nat. Comm. (2022), DOI: 10.1038/s41467-022-32744-9)). MDep_Ecoli heterologously expresses Mdh Bm MGA3.

The *E. coli* strain Screen_Ecoli, having the CCOS accession number CCOS 2134, which lacks the canonical formaldehyde detoxification pathway *(ΔfrmRAB),* was used to screen different methanol dehydrogenases for overoxidation of methanol to formate *in vivo.*

### Formate production by MEcoli_ref_2 during growth on methanol in a bioreactor

Supernatant samples were derived from bioreactor runs of MEcoli_ref_2 and analyzed by LC-MS.

### L-lactic acid, and itaconic acid production and formate side-production by MEcoli_ref_2 from methanol in shake flasks

Production of L-lactic acid and itaconic acid was performed as previously described (Reiter, M. A. et al.; Nat. Catal. 7, 560-573 (2024); DOI10.1038/s41929-024-01137-0). The cryogenic glycerol stocks of MEcoli_ref_2 transformed with pl_lac (lactic acid production) and MEcoli_ref 2 transformed with pl_ita (itaconic acid production) were inoculated in 20 ml volume of M9 medium supplemented with 500 mM methanol, 0.1 g L⁻¹ yeast extract and chloramphenicol in baffled 100 ml shake flasks. The cultures were incubated at 37 °C, 220 round per minute (r.p.m.)., 25 mm throw. In the exponential growth phase, eight replicate 20 ml cultures in M9 medium containing 500 mM methanol were inoculated to an initial OD600 of 0.05. At OD600 ≈ 1.2 (pl_lac) and OD600 ≈ 1.0 (pl_ita), production was induced by transferring the cells to fresh medium containing 500 nM anhydrotetracycline (atc). For the transfer, the cultures were centrifuged for 7 min at 3,220 g, the supernatant discarded, and the cells resuspended in fresh medium. Subsequently, the cultures were incubated in the dark at 37°C, 180 r.p.m., 50 mm throw. For supernatant sampling, 500 µL of culture were centrifuged for 1min at 11'000g, 450 µL of supernatant transferred to a fresh tube and stored at -20 °C until further analysis. Formate and itaconic acid were quantified by LC-MS, L-lactic acid by a commercially available enzymatic kit.

### Quantification of L-lactic acid

L-lactic acid in the culture supernatant was quantified by a commercially available enzyme kit (L-Lactic Acid (L-Lactate) Assay Kit, K-LATE, Megazyme) according to the manufacturer protocol.

### Quantification of formate and itaconic acid by LC-MS

Organic acids in the culture supernatant were derivatized together with an internal standard. 200 µM sodium propionate was used as internal standard for the simultaneous quantification of formate and itaconic acid, and for the determination of ¹²C/¹³C formate ratio in the isotopic tracer experiment displayed in Figure 1C. When only formate was quantified, 1 mM of isotopically labelled ¹³C formate was used as internal standard.

Samples were diluted 20-fold in 50% (v/v) acetonitrile and 34 mM 3-nitrophenylhydrazine and 21 mM N-(3-dimethylaminopropyl)-N'-ethylcarbodiimide were added. Samples were incubated at 40 °C with continuous shaking for 1h to facilitate the derivatization reaction. The reaction was quenched by the addition of 0.02% (v/v) trifluoroacetic acid and diluted 10-fold in 50% (v/v) acetonitrile. Derivatized samples were analyzed using UPLC with a C18 column with isobutyl side chains and tetramethylsilane end capping (Kinetex Core-Shell Technology XB-C18: 2.1× 50 mm, 1.7 µm particle size, 100 Å pore size, Phenomenex) coupled to a hybrid quadrupole-orbitrap mass spectrometer. Solvents were 0.1% (v/v) formic acid in ultrapure water (solvent A) and in acetonitrile (solvent B).

For metabolite separation, the following gradient was used at a constant flow rate of 500 µl min⁻¹: 98% solvent A linearly decreased to 5% in 3 min, then held at 5% A for 2 min, then linearly increased to 98% in 0.3 min and held at 98% for a final 2 min. Fourier-transform MS was performed in negative mode with a spray voltage of -3.0 kV, a capillary temperature of 275 °C, an S-lens radiofrequency level of 50, an auxiliary gas flow rate of 20 a.u. and an auxiliary gas heater temperature of 350 °C. Mass spectra were recorded as centroids at a resolution of 35,000 at an m/z of 200 with a mass range of 150-1,000 m/z and a scan rate of ^{~}4 Hz in full scan mode. 2 µl of sample was injected. Organic acids were quantified using pure formic acid or itaconic acid as external standards diluted in M9 medium derivatized with internal standard as described above. Metabolite peaks were extracted via a targeted approach using the external standard to define retention time-m/z peak windows applying an m/z tolerance of 10 ppm. Integration was performed using trapezoid integration. The ¹³C formate internal standard was corrected for the natural abundance of labelled formate in the sample and of labelled derivatization reagent.

### Adaptation of methanol-dependent strains to growth without yeast extract

The methanol-dependent ΔfrmAΔtpiA and *ΔfrmRABΔtpiA* strains require yeast extract as growth accelerator, which can be omitted after a couple of serial passages. For the adaptation of a strain to growth without yeast extract, cells were adapted in different conditions. Concentrations applied were methanol (500 mM for strains expressing Mdh2 CT4-1 Cn; 750 mM for strains expressing Mdh Bm MGA3 or Mdh Bm PB1), 20 mM pyruvate, 0.1 g L⁻¹ yeast extract in 20 mL M9 medium also containing, if indicated, Carb or Amp, Sm, IPTG. 100 mL baffled shake flasks were used as vessels. The first passage was inoculated from a cryogenic glycerol stock. Cultures were incubated at 37°C using a Multitron shaker at 220 r.p.m., 25 mm throw.

Once cells reached stationary phase, cultures were diluted. Passaging was repeated until growth without yeast extract was observed and the adapted culture was stored as cryogenic glycerol stock (25% glycerol) at -80°C until further analysis.

### Formate production during methanol-dependent growth

The cryogenic glycerol stock of the adapted methanol-dependent ΔfrmAΔtpiA strain expressing the RuMP cycle genes *mdh2* CT4-1 H165N Cn, *hps* Mf, and *phi* Mf (plasmids were isolated from an evolutionary stage between MEcoli_ref_1 and MEcoli_ref_239) were streaked out on a M9 minimal medium agar plate containing 500 mM methanol, 20 mM pyruvate, Sm, Amp and incubated at 37°C until colonies were formed. Four single colonies were inoculated in 20 mL M9 medium containing 500 mM methanol, 20 mM pyruvate, 0.1 g L⁻¹ yeast extract, Amp, Sm, IPTG in 100 mL baffled shake flasks and incubated at 37°C using a Multitron shaker at 220 r.p.m., 25 mm throw. Once cells reached late exponential/stationary phase, they were diluted by pipetting the required volume of culture into fresh medium with the same composition but without yeast extract. Once cells reached exponential phase, they were diluted again for the main culture. For supernatant sampling, 500 µL of culture were centrifuged for 1min at 11'000g, 450 µL of supernatant transferred to a fresh tube and stored at -20 °C until analysis by LC-MS.

For the adapted methanol-dependent strain harboring the same plasmids but containing a deletion of the entire formaldehyde detoxification pathway operon (*ΔfrmRABΔtpiA*)*,* the same procedure was followed but there was only 1 preculture and it was directly inoculated from a cryogenic glycerol stock.

For the adapted methanol-dependent *ΔfrmAΔtpiA* strain expressing *mdh* Bm MGA3, *hps* Mf, and *phi* Mf, the same procedure was followed but the preculture originated from the seventh passage of lineage 1 of its adaptation to growth without yeast extract and were passaged another 5 times until formate in the supernatant was quantified.

### Isotopic labelling experiment to determine origin of formate during methanol-dependent growth

The preculture handling and conditions were the same as described for the adapted methanol-dependent *ΔfrmAΔtpiA* strain expressing the RuMP cycle genes *mdh2* CT4-1 H165N Cn, *hps* Mf, and *phi* Mf in the previous paragraph with the following exceptions: 500 mM ¹³C instead of ¹²C methanol was used for the 2^{nd} preculture and the main culture; the culture volume was 10 mL; for the passaging from the 1^{st} to the 2^{nd} preculture, cells were transferred by centrifugation of the required culture volume, discarding the supernatant, and solution of the cell pellet in the fresh medium. Supernatant samples were also analyzed by LC-MS.

### Heterologous expression of enzymes

E. coli BL21-Gold (DE3) cells containing the expression vector were grown in 200 mL LB medium supplemented with Carb in 2 L baffled shake flasks at 37°C using a Multitron shaker at 220 r.p.m., 25mm throw. At an OD600 of about 0.7 (mid-exponential phase) the expression was induced with 0.3 mM IPTG and the culture was incubated overnight (about 16 h) at 16°C. The cells were harvested by centrifugation (11'000g, 10 min, 4°C) and either stored at -20°C or directly used for purification.

### Purification of Methanol dehydrogenases

Cell pellets were resuspended using 25 mL of buffer A (20 mM Tris, 20 mM NaCl, 2 mM DTT, pH 7.4) supplemented with Roche complete EDTA-free protease inhibitor cocktail. Cell lysis was performed by sonication (6 mm sonication probe, amplitude 30, process time 4 min, impulse time 5 s followed by 25 s cooling, Q700 sonicator) and cell debris was removed by centrifugation (18'000 x g, 40 min, 4°C).

As first chromatographic step to obtain untagged *mdh2* CT4-1 Cn and *mdh* Bm MGA3, the lysate was purified using a 1 mL HiTrap Q HP anion exchange column by applying a linear gradient of buffer A to buffer B (20 mM Tris, 1 M NaCl, 2 mM DTT, pH 7.4). The gradient was run at 1 mL/min from 0% B to 60% B in 30 min. The collected fractions were analyzed by SDS-PAGE, and fractions containing Methanol dehydrogenase were pooled and concentrated using 50 kDa centrifugal filter units.

As a next step, the sample was purified by size exclusion chromatography using Superdex 200 Increase 10/300 GL column run with buffer C (50 mM phosphate buffer, 150 mM NaCl, 5 mM MgSO₄, pH 7.4). The Methanol dehydrogenase-containing fractions were collected and concentrated. Protein concentration was determined by absorption measurement at 280 nm using a NanoDrop spectrophotometer and the calculated extinction coefficient of 21430 M⁻¹ cm⁻¹ (Mdh2 CT4-1 Cn) or 11920 M⁻¹ cm⁻¹ (Mdh Bm MGA3), respectively. The enzymes were stored at 4°C.

### In vitro enzyme assays

Activity assays were performed in cuvettes using a Cary 50 Bio UV-Visible spectrophotometer. Methanol dehydrogenase activity was assayed in reaction buffer (buffer C) supplemented with 12.3 µM Mdh2 CT4-1 Cn or 12.5 µM Mdh Bm MGA3 or 126.5 µM Chimera 1 and 5 mM NAD⁺ at 37°C. For the *in vitro* measurements involving Act, 3.1 µM Mdh Bm MGA3 and, if indicated, 2.3 µM activator protein (Act) was added. Assays were started by adding preheated (10 min) methanol or formaldehyde reaction buffer solution (10 µL) to preheated reaction buffer containing Mdh and NAD⁺, followed by mixing by pipetting up and down. The reaction was monitored by measuring the increase in NADH at 340 nm and the NADH calculated from its extinction coefficient (ε340 = 6220 M-1 cm-1).

To measure activity until equilibrium was reached, the assay was performed using a microplate reader and 1 mg mL⁻¹ enzyme. The equilibrium concentrations of formaldehyde, formate, and NADH were determined by the following methodologies: Formaldehyde was quantified by Nash assay. Formate was quantified by NMR. NADH was quantified by the absorbance of the reaction solution at 340 nm, using a standard curve obtained with a commercially available NADH standard.

In the initial range of in vitro reactions, the concentrations of formaldehyde or formate were often too small for reliable direct quantification. For this reason, NADH conversion was used as a readout for the initial activity as it correlates directly with the formation of either formaldehyde (from methanol) or formate (from formaldehyde) in the *in vitro* assay.

### Formate production of the ΔfrmRAB strain harboring plasmids encoding different NAD-dependent methanol dehydrogenases

From cryogenic glycerol stocks, the adapted *ΔfrmRAB* strains harboring an empty pSEVA424 plasmid backbone isolated from MEcoli_ref 2 or expressing different NAD-dependent Mdh enzymes were inoculated into 4 mL LB cultures in 12 mL plastic culture tubes and grown to stationary phase at 37°C using a Multitron shaker at 220 rounds per minute (r.p.m), 25 mm throw. Triplicate cultures were then inoculated in 20 mL M9 medium containing 20 mM pyruvate and 500 mM methanol to a starting OD600 of 0.05. 100 mL baffled shake flasks were used as vessels. Culture supernatants were sampled at 5, 22, 46, and 69 hours after inoculation. Samples for the quantification of formate by HPLC were generated by centrifugation of 500 µL of cell culture at 11.000 rcf, 4°C for 3 minutes. Next, 400 µL of the supernatant was carefully aspirated and subsequently stored in screw-cap tubes at -20°C until analysis by HPLC.

### Formate quantification by HPLC

Formate in the supernatant of the cultures for the methanol dehydrogenase screen for overoxidation of methanol was quantified via UPLC equipped with a Rezex ROA UPLC column (Rezex ROA-Organic Acid H+ (8%): 300 × 7.8 mm; Phenomenex) coupled to a variable wavelength detector VWD-3400 by monitoring the absorbance at 210 nm. 2.5 mM sulfuric acid in ultrapure water was used as solvent at a isocratic gradient and a flow rate of 600 µl min⁻¹. 20 µL of sample was injected. Peaks were identified and the peak areas were quantified based on an external standard curve of pure formic acid utilizing a standard dilution series in ultrapure water. Prior to injection, samples were diluted 1:1 (v/v) in ultrapure water and standards 1:1 (v/v) in M9 medium to exclude matrix effects.

### Phylogenetic analysis

Enzyme sequences aligned had the following Uniprot identifiers: Mdh Bm MGA3, Q6TV41; Mdh2 Bm MGA3, I3E2P9; Mdh3 Bm MGA3, I3E949; Mdh Bm PB1, I3DTP7; Mdh1 Bm PB1, I3DX19; Mdh2 Bm PB1, I3DVX6; Mdh2 Cn WT F8GNE5. The Mdh2 CT4-1 variant used in this study is A26V, A31V, A169V compared to the WT enzyme; Mdh Lx, A0A0K9F7R7; Mdh Gs, P42327.

The sequence alignment was performed using MUSCLE in the EMBL-EBI Job Dispatcher sequence analysis tools framework web tool74 and the phylogenetic tree was visualized by ETE375.

### Purification of Activator protein (Act)

His6-tagged Act Bm MGA3 was purified by resuspending cell pellets in 20 mL buffer A (50 mM phosphate, 300 mM NaCl, 20 mM imidazole, 2 mM DTT, pH 8) supplemented with Roche complete EDTA-free protease inhibitor. Cell lysis and clearance of cell debris was performed as described above.

Act was isolated from the lysate by affinity chromatography using a 1 mL HisTrap HP column and applying a linear gradient of buffer A to buffer B (50 mM phosphate buffer, 300 mM NaCl, 500 mM imidazole, 2 mM DTT, pH 8). The gradient was run at 1 mL/min from 0% B to 100% B in 25 min. Fractions containing Act were pooled and analyzed by SDS-PAGE. Buffer was exchanged to reaction buffer (50 mM phosphate buffer, 5 mM MgSO4, pH 7.4) by repeated concentration using 10 kDa centrifugal filter units and refills with reaction buffer until a dilution factor of at least 30'000 was achieved. Protein concentration was determined by absorption measurement at 280 nm using a NanoDrop spectrophotometer and the calculated extinction coefficient of 14'440 M⁻¹ cm⁻¹. The enzyme was stored at 4°C.

### Calculation of equilibrium concentrations

For a methanol overoxidizing Mdh, the oxidation of methanol to formaldehyde and of formaldehyde to formate was considered, while for a non-overoxidizing Mdh only the methanol to formaldehyde oxidation. Estimated ΔrG'° were derived from the eQuilibrator web tool for the conditions present in the *in vitro* assays (pH = 7.4, pMg = 2.3, ionic strength = 0.15M) and are 32 kJ/mol and -49.6 kJ/mol. The initial concentration of methanol is 500 mM and of NAD⁺ 5 mM. Equilibrium concentrations of methanol [MeOH], formaldehyde [FALDH], formate [FA], [NAD⁺] and [NADH] were derived from the following non-linear equation system for overoxidizing Mdh's: ${k}_{1} = \frac{\left[FALDH\right] ∗ \left[NADH\right]}{\left[MeOH\right] ∗ \left[{NAD}^{+}\right]}$ (1) ${k}_{2} = \frac{\left[FA\right] ∗ \left[NADH\right]}{\left[FALDH\right] ∗ \left[{NAD}^{+}\right]}$ (2) $500 = \left[MeOH\right] + \left[FALDH\right] + \left[FA\right]$ (3) $\left[NADH\right] = \left[FALDH\right] + 2 ∗ \left[FA\right]$ (4) $5 = \left[{NAD}^{+}\right] + \left[NADH\right]$ (5)

For non-overoxidizing Mdh's, the following non-linear equation system was solved: ${k}_{1} = \frac{\left[FALDH\right] ∗ \left[NADH\right]}{\left[MeOH\right] ∗ \left[{NAD}^{+}\right]}$ 91) $\begin{matrix}500 = \left[MeOH\right] + \left[FALDH\right] \\ \left[NADH\right] = \left[FALDH\right]\end{matrix}$ (6) $5 = \left[{NAD}^{+}\right] + \left[NADH\right]$ (5)

### Formaldehyde quantification by Nash assay

To quantify formaldehyde in samples from *in vitro* assays, 20 µL sample (collected at equilibrium, after 180 min) was taken from the assay mixture and mixed with 20 µL Nash reagent (2 M ammonium acetate, 0.05 M acetic acid, 0.02 M acetylacetone). Following an incubation of 40 min at 37°C, absorbance at 412 nm was measured using a microplate reader. Formaldehyde concentrations were calculated with a standard curve obtained by measuring the absorbance at 412 nm of known formaldehyde concentrations.

### Methanol, formaldehyde and formate quantification by NMR

90 µl of reaction mixture were mixed with 20 µl D₂O containing 10 mM DSS-D6 (98 %). 80 µl of 20 mM sodium acetate buffer and 10 µl of 1M acetic acid were added to acidify the sample. This mixture resulted in a final DSS proton concentration of 1000 µM. The prepared solutions were transferred to 3 mm NMR tubes, suitable for use with the SampleJet system.

NMR data were acquired at 298.0 K at a 600 MHz Bruker AVIII Avance HD NMR spectrometer equipped with a CP-QCI-1H/19F-13C/15N-2H 05 Z cryoprobe. The system included a SampleJet sample handling robot for automation, with the samples stored at 278 K between the experiments.

### 1D ¹H Experiments

The experiments were acquired with 65536 complex data points and 64 transients. The pre-scan relaxation delay was set to 4.0 s. The solvent signal was suppressed by applying pre-saturation during the pre-scan delay and by applying a short (10 ms) NOE mixing period followed by a z-gradient before the final π/2 pulse. Data were zero-filled to 131072 points, and a squared cosine window function was applied. The resulting spectra were baseline-corrected before peak integration. Peak integrals of metabolites were normalized to the integral of DSS at 0 ppm for concentration determination.

### 1D ¹³C Experiments

The experiments were acquired with 53056 complex data points and 6144 transients. The pre-scan relaxation delay was set to 2.0 s. The carrier frequency was set to 97 ppm and the spectral width to 251 ppm. The 1H channel was saturated during the pre-scan delay to enhance the ¹³C signal due to heteronuclear nuclear Overhauser enhancement. For 1H broadband decoupling during the acquisition and 1H saturation during the pre-scan delay, the WALTZ-6582,83 scheme was applied. Data were zero-filled to 131072 points, and a squared cosine window function was applied. The resulting spectra were baseline-corrected before peak integration.

Data acquisition and processing were performed using TopSpin 3.6. Formate was quantified using pure formic acid as external standard diluted in M9 medium.

The nucleotide sequences encoding the Chimera enzymes 1 and 2 were synthesized by Twist Bioscience and cloned into a pET16 expression vector using Gibson assembly.

### Whole cell enzyme assay to test overoxidation of methanol to formate

pET16 expression vector were transformed into BL21(DE3) Gold cells with an interrupted formaldehyde detoxification pathway Δ*frm*A and stocked as cryogenic glycerol stock. Cryogenic glycerol stocks were streaked-out onto LB plates containing carbenicillin and a single colony inoculated into 20 mL LB containing carbenicillin in a 100mL baffled shakeflask. Cells were incubated at 37 °C until the cultures reached an OD600 between 0.6 - 0.8 using a Multitron shaker at 220 r.p.m., 25 mm throw. When the target density was reached, cultures were put at 16 °C for 30 min using a Multitron shaker at 220 r.p.m., 25 mm throw and subsequently, protein expression was induced by the addition of 0.3 mM IPTG. Cultures were incubated at 16 °C overnight for protein expression. The next day was protein expression confirmed by SDS PAGE and three times 1.2 mL of culture were transferred into a 24-well deep well plate per strain. 800 mM 13C methanol was added to each well and the cells incubated at 37 °C. After 10h, the supernatant was sampled by centrifugation of 250 µL culture for 1 min at 11'000 g and 220 µL of supernatant were transferred to a fresh tube. Samples were stored at -20 °C until methanol, formaldehyde, and formate quantification by NMR.

## Claims

1. A recombinant non-naturally occurring methanol-dependent or fully methylotrophic microorganism,
which heterologously expresses or over-expresses a polypeptide having methanol dehydrogenase (Mdh) activity,
wherein the initial maximum velocity of the oxidization of formaldehyde to formate reaction (VₘₐₓCH₂O) catalysed by said polypeptide having Mdh activity measured as [nM_{NADH} second⁻¹ µM_{enzyme}⁻¹], is less than 1, or less than 0.1 or 0.

2. The recombinant microorganism of claim 1, which further expresses or overexpresses a polypeptide having 3-hexulose-6-phosphate synthase (Hps) activity, and a polypeptide having a 6-phospho 3-hexuloisomerase (Phi) activity, and/or which does not comprise, or which comprises deletions or reductions of expression of, or eliminations or reductions of activity of a polypeptide having triose-phosphate-isomerase activity and of a polypeptide having glutathione-dependent formaldehyde dehydrogenase activity.

3. The recombinant microorganism of claim 1 or 2, wherein the polypeptide having a Mdh activity has at least 70%, 75%, 80%, 85%, 90%, 95%, 98% or 99% sequence identity to SEQ ID NO 1.

4. The recombinant microorganism of any of claims 1 to 3, wherein the polypeptide having a Mdh activity is selected from a group consisting of SEQ ID NO 1, SEQ ID NO 2, SEQ ID NO 3, SEQ ID NO 4, SEQ ID NO 5, SEQ ID NO 6.

5. The recombinant microorganism of any of claims 1 to 4, wherein the polypeptide having a Mdh activity comprises a sequence having at least 70%, 80%, 90%, 95%, 98% or 99% sequence identity to SEQ ID NO 13 and/or comprising a sequence having at least 60%, 70%, 80%, 90%, 95%, 98% or 99% sequence identity to SEQ ID NO 14, wherein said sequences, if both present in the polypeptide are spaced at least 40 residues apart, for example 40 to 50 or 40 to 45 residues apart.

6. The recombinant microorganism of any of claims 1 to 5, wherein the polypeptide having a Mdh activity comprising an N-terminal part having at least 70%, 75%, 80%, 85%, 90%, 95%, 98% or 99% sequence identity to the N-terminal part containing amino acids 1-178 of Mdh Bm MGA3 (SEQ ID NO 1).

7. The recombinant microorganism of any of claims 1 to 6, expressing or over-expressing a polypeptide having a Nudix hydrolase Act (SEQ ID NO 12) activity.

8. The recombinant microorganism of any of claims 1 to 7, expressing or over expressing all enzymes required for a complete ribulose monophosphate (RuMP) cycle, consisting of a polypeptide having methanol dehydrogenase activity, such as Mdh, a polypeptide having 3-hexulose 6-phosphate synthase, such as Hps, a polypeptide having 6-phospho 3-hexuloisomerase activity, such as Phi, a polypeptide having glucose-6-phosphate isomerase activity, such as Pgi, a polypeptide having transketolase activity, such as TktA, a polypeptide having ribulose-phosphate 3-epimerase, such as Rpe, a polypeptide having ribose-5-phosphate isomerase activity, such as RpiA, a polypeptide having transaldolase activity, such as TalA, a polypeptide having NADP⁺-dependent glucose-6-phosphate dehydrogenase, such as Zwf, a polypeptide having 2-keto-3-deoxygluconate 6-phosphate/2-keto-4-hydroxyglutarate aldolase activity, such as Eda, and a polypeptide having a phosphogluconate dehydratase, such as Edd.

9. The recombinant microorganism of any of claims 1 to 8, which is a recombinant non-naturally occurring methanol-dependent or fully methylotrophic bacterial organism, for example *Escherichia, such as E. coli, Bacillus,* such as *B. subtilis, Clostridium, Enterobacter, Klebsiella, Mannheimia, Pseudomonas,* such as *P. putida, Acinetobacter, Shewanella, Paracoccus, Ralstonia,* such as *R. eutropha, Geobacter, Zymomonas, Acetobacter, Geobacillus, Lactococcus, Streptococcus, Lactobacillus, Corynebacterium,* such as C. *glutamicum, Streptomyces, Proprionibacterium, Synechocystis, Synechococcus, Cyanobacteria, Chlorobi,* and *Deinococcus.*

10. The recombinant microorganism of any of claims 1 to 9, which is the designated *Escherichia coli* MDep_Ecoli having CCOS accession number CCOS 2133, or an *Escherichia coli* strain having a doubling time and product profile of CCOS deposit accession number CCOS 2133.

11. The recombinant microorganism of any of claims 1 to 10, which is
- the designated *Escherichia coli* MEcoli_ref_1 having CCOS accession number CCOS 2032,
- the designated *Escherichia coli* MEcoli_ref_2 having CCOS accession number CCOS 2080,
- the designated *Escherichia coli* SM1 having ATCC accession number PTA-126783,
- or an *Escherichia coli* strain having a doubling time and product profile of CCOS deposit accession number CCOS 2032, of CCOS deposit accession number CCOS 2080, or of ATCC accession number PTA-126783.

12. A recombinant polypeptide having Mdh activity comprising
- an N-terminal part with at least 70%, 75%, 80%, 85%, 90%, 95%, 98% or 99% sequence identity to the N-terminal part containing amino acids 1-178 of Mdh Bm MGA3 (SEQ ID NO 1), or
- a sequence having at least 70%, 80%, 90%, 95%, 98% or 99% sequence identity to SEQ ID NO 13 and/or comprising a sequence having at least 70%, 80%, 90%, 95%, 98% or 99% sequence identity to SEQ ID NO 14, wherein said sequences, if both present in the polypeptide are spaced at least 40 residues apart, for example 40 to 50 or 40 to 45 residues apart.

13. An expression vector or a cell comprising a nucleic acid sequence encoding a recombinant polypeptide comprising
- an N-terminal part with at least 70%, 75%, 80%, 85%, 90%, 95%, 98% or 99% sequence identity to the N-terminal part containing amino acid residues 1-178 of Mdh Bm MGA3 (SEQ ID NO 1), or
- a sequence having at least 70%, 80%, 90%, 95%, 98% or 99% sequence identity to SEQ ID NO 13 and/or comprising a sequence having at least 70%, 80%, 90%, 95%, 98% or 99% sequence identity to SEQ ID NO 14, wherein said sequences, if both present in the polypeptide are spaced at least 40 residues apart, for example 40 to 50 or 40 to 45 residues apart.

14. A method for producing or for increasing production of a microbial metabolite or product, comprising the steps of growing the recombinant microorganism of any of claims 1 to 11 on a C1 compound, for example methanol, and obtaining the metabolite or product.

15. The method of claim 14, wherein the microbial metabolite or product is selected from a group consisting of 2-carbon compounds; 3-carbon compounds, such as lactate, or lactic acid; 4-carbon compounds, such as isobutanol, butanol, or 1,4-butanediol; 5-carbon compounds, such as n-pentane, glutamate; higher carboxylic acids, alcohols of higher carboxylic acids; polyhydroxyalkanoates, such as polyhydroxybutyrate (PHB); taxadiene; itaconate; speciality chemicals; aromatic compounds; vitamins; derivatives or precursors of vitamines; carotenoids; amino acids; and sugars, such as deoxyribose.
